# EUROPÄISCHE PATENTSCHRIFT

(11) **EP 0 822 933 B1**
(45) Veröffentlichungstag und Bekanntmachung des Hinweises auf die Patenterteilung: **24.10.2001**
(21) Anmeldenummer: 96909998.5
(22) Anmeldetag: 19.03.1996
(51) Int. Cl.: C07D 295/13, C08G 18/20

(54) **VERFAHREN ZUR HERSTELLUNG VON PIPERIDINOPENTANAMINEN**
PROCESS FOR PRODUCING PIPERIDINOPENTANAMINES
PROCEDE DE PREPARATION DE PIPERIDINOPENTANAMINES

(30) Priorität: 26.04.1995 CH 119095
(43) Veröffentlichungstag der Anmeldung: 11.02.1998
(60) Teilanmeldung: 00127106.3
(73) Patentinhaber: Lonza AG, 3930 Visp (CH)
(72) Erfinder: HEVELING, Josef, CH-3904 Naters (CH); GERHARD, Andreas, CH-3930 Visp (CH)
(74) Vertreter: Weinhold, Peter, Dr.rer.nat. Dipl.-Chem.
(86) Internationale Anmeldenummer: EP9601179
(87) Internationale Veröffentlichungsnummer: WO9633986

(56) Entgegenhaltungen:
- EP-A- 0 495 249
- EP-A- 0 696 580
- DE-A- 4 033 632
- FR-A- 2 085 965
- US-A- 2 949 431
- US-A- 5 268 470
- CHEMICAL ABSTRACTS, vol. 90, no. 17, 23.April 1979 Columbus, Ohio, US; abstract no. 137153, XP002006813 & BULL. ACAD. POL. SCI., SER. SCI. CHIM., Bd. 26, Nr. 8, 1978, Seiten 575-582,
- J. APPLIED POLYMER SCIENCE, Bd. 41, Nr. 9/10, 1990, Seiten 2059-2065, XP000204335 D. KATSAMBERIS, S. P. PAPPAS: "Catalysis of Isocyanate-Alcohol and Blocked Isocyanate-Alcohol Reactions by Amidines"

## Beschreibung

Die vorliegende Erfindung betrifft ein Verfahren zur Herstellung von Piperidinopentanaminen, sowohl einzeln als auch als Gemisch, aus 2-Methylglutaronitril, namentlich dem 2-Methyl-5-(3-methylpiperidino)-pentanamin und dem isomeren 4-Methyl-5-(3-methylpiperidino)-pentanamin, die sich durch die gemeinsame Formel 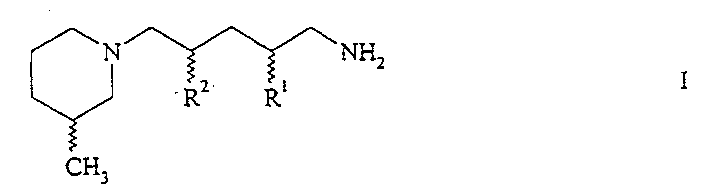 darstellen lassen. Hierin steht jeweils einer der beiden Reste R¹ und R² für Wasserstoff und der andere für Methyl. Im Fall des 2-Methyl-5-(3-methylpiperidino)-pentanamins (Ia) ist R¹ = Methyl und R² = H, im Fall des 4-Methyl-5-(3-methylpiperidino)pentanamins (Ib) ist R¹ = H und R² = Methyl. Beide Verbindungen können jeweils in vier verschiedenen stereoisomeren Formen (zwei Diastereomerenpaare) vorliegen. Hier und im folgenden stehen die Formeln und die zugehörigen Verbindungsnamen jeweils für alle möglichen Stereoisomeren.

Die Verbindungen der Formel I können als Katalysatoren für die Herstellung von Polyurethanen aus Polyisocyanaten und Polyolen verwendet werden.
Für die Herstellung von Polyurethanen werden üblicherweise Stickstoffbasen, insbesondere tertiäre Amine wie beispielsweise 1,4-Diazabicyclo[2.2.2]octan (DABCO® ), cyclische Amidine wie beispielsweise 1,8-Diazabicyclo[5.4.0]undec-7-en ("DBU") oder auch Guanidin-derivate wie Tetramethylguanidin als Katalysatoren eingesetzt. Für die Eignung als Katalysator sind u.a. die Basizität, Löslichkeitsverhalten und Flüchtigkeit massgebend. Während die Basizität in erster Linie die katalytische Wirksamkeit bestimmt, sind Löslichkeitsverhalten und Flüchtigkeit für die Verarbeitungseigenschaften ebenfalls von Bedeutung. Zu hohe Flüchtigkeit beispielsweise kann durch Freisetzung in die Atmosphäre bei der Herstellung der Polyurethane oder ihrem Gebrauch zur Belästigung oder Gefährdung von Personen führen.
Die DE-A-40 33 632 beschreibt Morpholinopropanamine, ein Verfahren zu deren Herstellung sowie deren Verwendung als Katalysatoren bei der urethanbildenden Umsetzung eines Polyols mit einem Polyisocyanat. Die FR-A-2 085 965 offenbart heterocyclische Alkylaminoverbindungen als Katalysatoren bei der Herstellung von Polyurethanen.
Aufgabe der vorliegenden Erfindung war es, neue Amine bereitzustellen, die als Katalysator zur Herstellung von Polyurethanen geeignet sind, günstige Gebrauchseigenschaften aufweisen und einfach und kostengünstig herzustellen sind.

Erfindungsgemäss wird die Aufgabe durch das Herstellungsverfahren nach Anspruch 1 gelöst.

Es wurde gefunden, dass durch Umsetzung des bei der Adiponitrilherstellung als Nebenprodukt anfallenden 2-Methylglutaronitrils mit Wasserstoff in Gegenwart eines Katalysators in einer Stufe neben anderen Produkten ein Gemisch der beiden isomeren Methyl-5-(3-methylpiperidino)pentannitrile der allgemeinen Formel 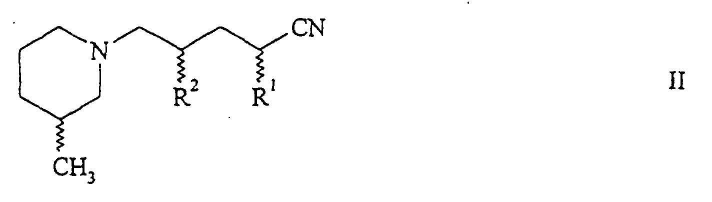 worin einer der beiden Reste R¹, R² Wasserstoff und der andere Methyl bedeutet, hergestellt und in einer zweiten Stufe in Gegenwart eines zweiten Katalysators zu dem Gemisch der beiden isomeren Methyl-5-(3-methylpiperidino)pentanamine Ia und Ib hydriert werden kann.

Das Isomerengemisch Ia/Ib kann gegebenenfalls destillativ oder chromatographisch in die beiden Strukturisomeren Ia und Ib aufgetrennt werden, wird aber vorzugsweise als solches verwendet.

Als Katalysator für die erste Stufe wird vorzugsweise ein Palladium-Trägerkatalysator eingesetzt, besonders bevorzugt ist Palladium auf Aluminiumoxid.

Die Umsetzung des 2-Methylglutaronitrils mit Wasserstoff wird vorzugsweise bei einer Temperatur von 100 bis 250 °C und einem Druck von 20 bis 70 bar durchgeführt.

Es hat sich als vorteilhaft erwiesen, dem 2-Methylglutaronitril etwas 3-Methylpiperidin zuzusetzen. Vorzugsweise wird das 3-Methylpiperidin in einer Menge von 1 bis 5 mol auf 1 mol 2-Methylglutaronitril zugegeben. Die Selektivität der Reaktion wird hierdurch wesentlich gesteigert. 3-Methylpiperidin kann nach bekannten Verfahren ebenfalls aus 2-Methylglutaronitril hergestellt werden (WO 90/00 546).

Die Umsetzung des 2-Methylglutaronitrils mit Wasserstoff wird vorzugsweise kontinuierlich in einem Festbettreaktor durchgeführt.

Die Hydrierung der Methyl-5-(3-methylpiperidino)pentannitrile II zu den Methyl-5-(3-methylpiperidinb)pentanaminen Ia/Ib wird vorteilhaft in Gegenwart von Ammoniak durchgeführt, um unerwünschte Nebenreaktionen zu unterdrücken.

Als Katalysatoren für diese zweite Stufe können grundsätzlich alle Katalysatoren eingesetzt werden, die sich für die Hydrierung von Nitrilen zu Aminen eignen, also beispielsweise Palladium, Platinum, Rhodium, Cobalt, Nickel oder Nickel-Borid.

Vorzugsweise wird als Katalysator für die zweite Stufe ein Rhodium-Trägerkatalysator eingesetzt, besonders bevorzugt ist Rhodium auf Aktivkohle.

Die Hydrierung der Methyl-5-(3-methylpiperidino)pentannitrile II an einem Rhodium-Trägerkatalysator wird vorzugsweise bei einer Temperatur von 5 bis 100 °C und einem Druck von 1 bis 50 bar durchgeführt.

Als Lösungsmittel für die zweite Stufe können die für die Hydrierung von Nitrilen zu Aminen üblichen Lösungsmittel eingesetzt werden. Vorzugsweise werden Alkohole wie beispielsweise Ethanol oder *tert*-Butylalkohol eingesetzt.

Die erfindungsgemässen Methyl-5-(3-methylpiperidino)pentanamine Ia/Ib können, wie bereits eingangs erwähnt, als Katalysatoren zur Herstellung von Polyurethanen eingesetzt werden.

Die nachfolgenden Beispiele verdeutlichen die Herstellung und Verwendung der erfindungsgemässen Verbindungen.

### Beispiele:

### Beispiel 1

In einen Reaktor (13 mm Ø) wurden 3 g Pd/Al₂O₃-Katalysator (1% Pd, Korngrösse 0,315-1,0 mm) eingefüllt. Der Reaktor wurde im Wasserstoffstrom (120 ml/min, bezogen auf Normaldruck) bei 50 bar auf die Reaktionstemperatur von 150 °C aufgeheizt. Dann wurde dem Wasserstoff eine Mischung aus 3-Methylpiperidin und 2-Methylglutaronitril im molaren Verhältnis von 2:1 zudosiert. Der Durchsatz betrug 2,1 g Edukt pro g Katalysator und Stunde. Das Produktgemisch nach Verlassen des Reaktors und Abtrennen des Wasserstoffs enthielt gemäss GC 44,8% 2(4)-Methyl-5-(3-methylpiperidino)pentannitril (Isomerengemisch), 30,2% 3-Methylpiperidin und 24,4% 1,5-Bis(3-methylpiperidino)-2-methylpentan sowie 0,6% nicht identifizierte Produkte.
Das Gemisch wurde über 260 h Reaktionsdauer gesammelt und anschliessend im Vakuum fraktioniert. Bei 87 °C/2,5 mbar ging das Methyl-5-(3-methylpiperidino)pentannitril in einer Reinheit von 99,5% (GC) über. Gemäss NMR-Analyse handelte es sich um ein Gemisch aus 85% 2-Methyl- und 15% 4-Methyl-Verbindung.

| Analytische Daten: | | | |
|---|---|---|---|
| C₁₂H₂₂N₂ Berechnet | C 74,2 | H 11,4 | N 14,4 |
| Gefunden | C 74,2 | H 11,6 | N 14,9 |

- ¹H NMR (CDCl₃, 400 MHz) δ: (Hauptkomponente): 1,40-2,85 (m, 16H, CH + CH₂);
1,32 (d, 3 H, C*H*₃―CH―CN);
0,86 (d, 3 H, Ring-CH₃).
- ¹³C NMR (CDCl₃, 100 MHz) δ: (Hauptkomponente): 123,01 (s); 62,20 (t); 58,28 (t); 54,12 (t);
33,13 (t); 32,23 (t); 31,19 (d); 25,62 (t);

### Beispiel 2

Es wurde verfahren wie in Beispiel 1 beschrieben, jedoch betrug die Reaktionstemperatur nur 125 °C.
Das Produktgemisch enthielt ca. 50% 2-Methyl-5-(3-methylpiperidino)pentannitril, 8% 4-Methyl-5-(3-methylpiperidino)pentannitril, 32% 3-Methylpiperidin, 6% 1,5-Bis(3-methylpiperidino)-2-methylpentan, 1% 2-Methylglutaronitril und 3% nicht identifizierte Produkte.

Das Gemisch wurde über 284 h Reaktionsdauer gesammelt und anschliessend im Vakuum fraktioniert. Bei 87 °C/2,5 mbar ging das Methyl-5(3-methylpiperidino)pentannitril in einer Reinheit von 99,3% (GC) über. Gemäss NMR-Analyse handelte es sich um ein Gemisch aus 86% der 2-Methyl- und 14% der 4-Methyl-Verbindung.

### Beispiel 3

In einen Reaktor (13 mm Ø) wurden 3 g eines Pd/MgCl₂/Al₂O₃-Katalysators (1% Pd, 1,2% Mg, Korngrösse 0,315-1,0 mm) eingefüllt. Der Reaktor wurde im Wasserstoffstrom (120 ml/min, bezogen auf Normaldruck) bei 50 bar auf die Reaktionstemperatur von 160 °C aufgeheizt. Dann wurde dem Wasserstoff 99,8%iges 2-Methylglutaronitril zudosiert. Der Durchsatz betrug 2,1 g Edukt pro g Katalysator und Stunde. Gemäss GC enthielt der Produktstrom 25,8% 2(4)-Methyl-5-(3-methylpiperidino)pentannitril (Isomerengemisch), 52,1% 1,5-Bis(3-methylpiperidino)-2-methylpentan, 3,7% 3-Methylpiperidin und 18,4% nicht identifizierte Produkte.
Die Selektivität der Reaktion zu den gewünschten Nitrilen war im Vergleich zu den Beispielen 1 und 2 (mit Zusatz von 3-Methylpiperidin) deutlich geringer.

### Beispiel 4

In 100 g trockenen *tert*-Butylalkohol wurde unter Kühlung Ammoniak eingeleitet, bis eine Konzentration von 13,7% NH₃ erreicht war. Ein 100 ml Autoklav mit magnetgetriebenem Rührer wurde mit Stickstoff gespült und vorgekühlt. Dann wurden 30 g der Lösung von Ammoniak in *tert*-Butylalkohol, 4 g Rhodium/Aktivkohle (5% Rh) und 30 g des Gemischs der Methyl-5-(3-methylpiperidino)pentannitrile aus Beispiel 1 eingefüllt. Der Autoklav wurde geschlossen und unter Rühren erwärmt. Bei Raumtemperatur wurden 10 bar Wasserstoff aufgepresst, dann wurde die Temperatur weiter auf 50 °C erhöht. Nach 6 h war gemäss GC ein Umsatz von 99,2% und eine Ausbeute von 87,3% erreicht.

Das Reaktionsgemisch wurde im Vakuum destilliert, wobei das Isomerengemisch 2(4)-Methyl-5-(3-methylpiperidino)pentanamin bei 104 °C/3 mbar in einer Reinheit von 99,5% erhalten wurde. Das Gemisch enthielt gemäss GC die 2-Methyl- und die 4-Methyl-Verbindung im Verhältnis 2,6:1.

| Analytische Daten: | | | |
|---|---|---|---|
| C₁₂H₂₆N₂ Berechnet | C 72,7 | H 13,2 | N 14,1 |
| Gefunden | C 72,9 | H 13,1 | N 14,6 |

- ¹H NMR (CDCl₃, 400 MHz) δ =: 0,6-0,8 (CH₃, 1 Methylen-H);
1,0-1,25 (NH₂, 1 Methylen-H)
1,30-1,85;
2,05;
2,27;
2,55;
2,65-2,80.

Die NMR-Spektren sind wegen des Vorliegens mehrerer diastereomerer Formen sehr komplex.

### Beispiele 5-8

Das Isomerengemisch 2-Methyl-5-(3-methylpiperidino)pentanamin/4-Methyl-5-(3-methylpiperidino)pentanamin (2,6:1) aus Beispiel 4 wurde als Polyurethan-Katalysator eingesetzt:

### Abkürzungen:

VL: Desmodur® VL von Bayer, aromatisches Diisocyanat mit ca. 32% -NCO
D550U: Desmophen® 550U, Polypropylenglykol von Bayer, trifunktionell mit 10,5%-OH
DBU: Diazabicyclo[5.4.0]undec-7-en
MMPPA: Gemisch aus 2-Methyl- und 4-Methyl-5-(3-methylpiperidino)pentanamin (2,6:1)

Als Vergleichskatalysator diente DBU. Desmophen® wurde mit dem Amin (DBU oder MMPPA) vorgelegt und gut gemischt. Es entstand eine Lösung. In Beispiel 7 und 8 wurde zusätzlich Wasser zugegeben. Es entstand eine Emulsion/Lösung. Eine eingewogene Menge Isocyanat (VL) wurde zum Zeitpunkt *t* = 0 unter heftigem Rühren zugesetzt. Es wurde der Zeitpunkt notiert,
- zu dem die Lösung nicht mehr trübe war,
- zu dem eine deutliche Erwärmung feststellbar war,
- zu dem die Mischung fest wurde.

Die Resultate (MMPPA im Vergleich zu DBU) gehen aus der folgenden Tabelle 1 hervor:

**Tabelle 1:**

| Bsp. Nr. | *t* [s] nicht mehr trübe | *t* [s] Erwärmung | *t* [s] fest | VL [g] | D550U [g] | DBU [g] | MMPPA [g] | H₂O [g] | Vol. [ml] | Bemerkungen (Farbe etc.) |
|---|---|---|---|---|---|---|---|---|---|---|
| 5 | 550 | 360 | 820 | 50,0 | 55,0 | 0,1 | - | - | | braun/ocker, hart |
| 6 | *295* | 145 | 550 | 50,0 | 55,0 | - | 0,1 | - | | ocker, hart |
| 7 | 200 | 180 | 1180 | 50,0 | 45,0 | 0,1 | - | 0,5 | 400 | ocker, Hartschaum |
| 8 | 175 | 150 | 960 | 50,0 | 45,0 | - | 0,1 | 0,5 | 300 | ocker, hart |

### Beispiele 9-12

Das Polymer aus den Beispielen 5 und 6 wurde zu feinen Flocken zerhobelt und jeweils 4 g davon in 45 g Ethanol aufgeschlämmt und täglich bewegt. Mit GC wurde bestimmt, wieviel des Katalysators nach einem Tag, nach einer Woche und nach drei Wochen aus dem Polymer ausgetreten war. Die Resultate (MMPPA im Vergleich zu DBU) gehen aus der folgenden Tabelle 2 hervor. Es zeigte sich, dass im Gegensatz zu DBU der neue Katalysator nicht eluiert wurde. Daraufhin wurde der Versuch mit einer grösseren Katalysatormenge wiederholt (Beispiele 11 und 12). Nach einem Tag konnte jetzt zwar MMPPA in der Lösung nachgewiesen werden, aber die Menge war wesentlich geringer als im entsprechenden Vergleichsversuch mit DBU, und sie erhöhte sich über die nächsten 3 Wochen praktisch nicht.

**Tabelle 2**

| Beispiel Nr. | Katalysator | Kat.-Menge [g] | 1 Tag [GC-Fläche] | 1 Woche [GC-Fläche] | 3 Wochen [GC-Fläche] |
|---|---|---|---|---|---|
| 9 | DBU | 0,1 | 3520 | 3580 | 7080 |
| 10 | MMPPA | 0,1 | 0 | 0 | 0 |
| 11 | DBU | 0,5 | 15800 | 21900 | 28200 |
| 12 | MMPPA | 0,5 | 580/1290* | 475/1350* | 590/1460* |

| | | | | | |
|---|---|---|---|---|---|
| * Die erste Zahl bezieht sich auf die 4-Methyl-Verbindung, die zweite auf die 2-Methyl-Verbindung | | | | | |

## Patentansprüche

1. Verfahren zur Herstellung von Methyl-5-(3-methylpiperidino)pentanaminen der allgemeinen Formel 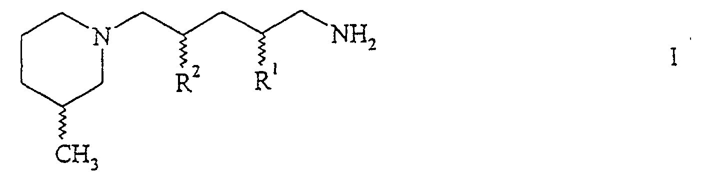 worin einer der beiden Reste R¹, R² Wasserstoff und der andere Methyl bedeutet, oder deren Gemischen, **dadurch gekennzeichnet, dass** in einer ersten Stufe 2-Methylglutaronitril, gegebenenfalls unter Zusatz von 3-Methylpiperidin, in Gegenwart eines ersten Katalysators mit Wasserstoff zu einem Gemisch der isomeren Methyl-5-(3-methylpiperidino)pentannitrile der allgemeinen Formel 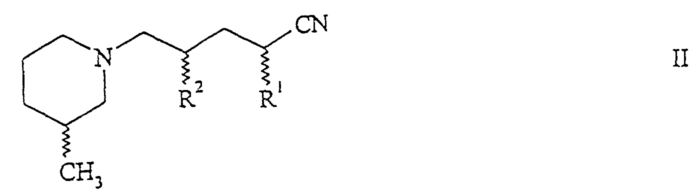 worin R¹ und R² die oben genannte Bedeutung haben, umgesetzt wird, welches in einer zweiten Stufe in Gegenwart eines zweiten Katalysators zu einem Gemisch der isomeren Methyl-5-(3-methylpiperidino)pentanamine der Formeln 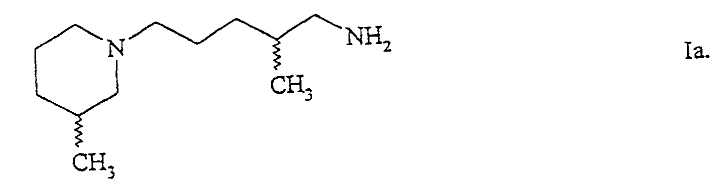 und 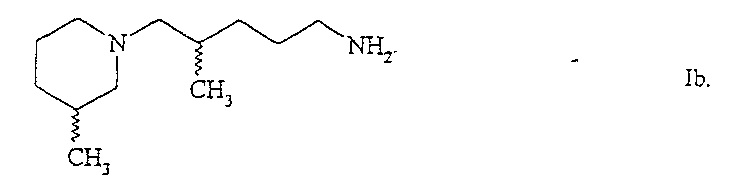 hydriert und gegebenenfalls in die beiden Strukturisomeren aufgetrennt wird.

2. Verfahren nach Anspruch 1, **dadurch gekennzeichnet, dass** als erster Katalysator ein Palladium-Trägerkatalysator eingesetzt wird.

3. Verfahren nach Anspruch 2, **dadurch gekennzeichnet, dass** als Palladium-Trägerkatalysator Palladium auf Aluminiumoxid eingesetzt wird.

4. Verfahren nach Anspruch 2 oder 3, **dadurch gekennzeichnet, dass** die Umsetzung des 2-Methylglutaronitrils mit Wasserstoff bei einer Temperatur von 100 bis 250 °C und einem Druck von 20 bis 70 bar durchgeführt wird.

5. Verfahren nach einem oder mehreren der Ansprüche 1 bis 4, **dadurch gekennzeichnet, dass** dem 2-Methylglutaronitril die 1 bis 5fache molare Menge 3-Methylpiperidin zugesetzt wird.

6. Verfahren nach einem oder mehreren der Ansprüche 1 bis 5, **dadurch gekennzeichnet, dass** die Umsetzung des 2-Methylglutaronitrils mit Wasserstoff kontinuierlich in einem Festbettreaktor durchgeführt wird.

7. Verfahren nach einem oder mehreren der Ansprüche 1 bis 6, **dadurch gekennzeichnet, dass** die Hydrierung der Methyl-5-(3-methylpiperidino)pentannitrile (II) in Gegenwart von Ammoniak durchgeführt wird.

8. Verfahren nach einem oder mehreren der Ansprüche 1 bis 7, **dadurch gekennzeichnet, dass** als zweiter Katalysator ein Rhodium-Trägerkatalysator eingesetzt wird.

9. Verfahren nach Anspruch 8, **dadurch gekennzeichnet, dass** als Rhodium-Trägerkatalysator Rhodium auf Aktivkohle eingesetzt wird.

10. Verfahren nach Anspruch 8 oder 9, **dadurch gekennzeichnet, dass** die Hydrierung der Methyl-5-(3-methylpiperidino)pentannitrile bei einer Temperatur von 5 bis 100 °C und einem Druck von 1 bis 50 bar durchgeführt wird.

## Claims

1. A process for preparing methyl-5-(3-methylpiperidino)pentanamines of the general formula 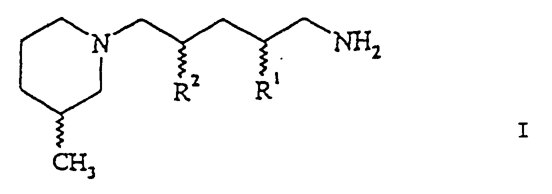 in which one of the two groups R¹, R² represents hydrogen and the other represents methyl, or their mixtures, **characterised in that** in a first step 2-methylglutaronitrile, optionally with the addition of 3-methylpiperidine, is reacted with hydrogen in the presence of a first catalyst to give a mixture of the isomers of methyl-5-(3-methylpiperidino)pentanonitrile of the general formula 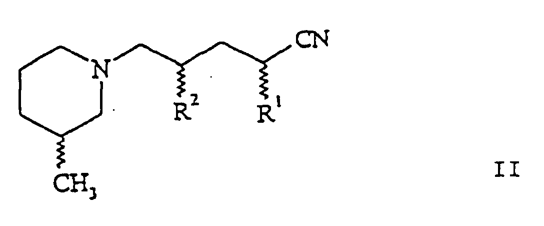 in which R¹ and R² are defined in the same way as above, which is hydrogenated in a second step in the presence of a second catalyst to give a mixture of the isomers of methyl-5-(3-methylpiperidino)pentanamines of the formulae 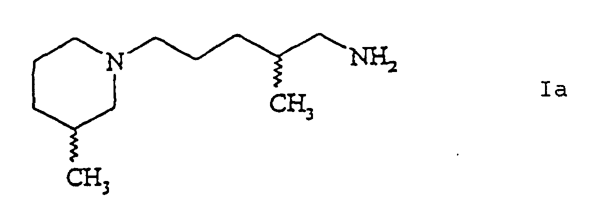 and 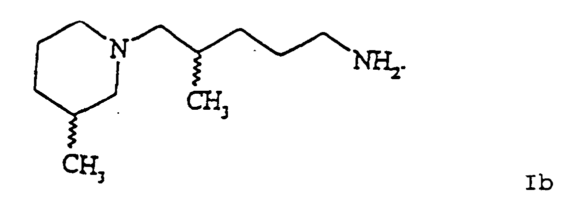 and optionally separated into the two structural isomers.

2. A process according to Claim 1, **characterised in that** a palladium supported catalyst is used as the first catalyst.

3. A process according to Claim 2, **characterised in that** palladium on aluminium oxide is used as a palladium supported catalyst.

4. A process according to Claim 2 or 3, **characterised in that** the reaction of 2-methylglutaronitrile with hydrogen is performed at a temperature of 100 to 250°C and a pressure of 20 to 70 bar.

5. A process according to one or more of Claims 1 to 4, **characterised in that** 1 to 5 times the molar amount of 3-methylpiperidine is added to the 2-methylglutaronitrile.

6. A process according to one or more of Claims 1 to 5, **characterised in that** the reaction of 2-methylglutaronitrile with hydrogen is performed continuously in a fixed bed reactor.

7. A process according to one or more of Claims 1 to 6, **characterised in that** hydrogenation of methyl-5-(3-methylpiperidino)pentanonitrile (II) is performed in the presence of ammonia.

8. A process according to one or more of Claims 1 to 7, **characterised in that** a rhodium supported catalyst is used as a second catalyst.

9. A process according to Claim 8, **characterised in that** rhodium on active carbon is used as a rhodium supported catalyst.

10. A process according to Claim 8 or 9, **characterised in that** the hydrogenation of methyl-5-(3-methylpiperidino)pentanonitrile is performed at a temperature of 5 to 100°C and a pressure of 1 to 50 bar.

## Revendications

1. Procédé de préparation de méthyl-5-(3-méthylpipéridino)pentanamines de formule générale 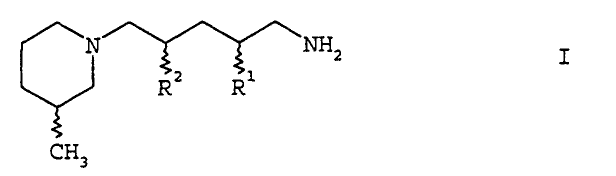 où l'un des deux restes R¹, R² représente l'hydrogène et l'autre représente méthyle, ou de leurs mélanges, **caractérisé en ce que**, dans une première étape, le 2-méthylglutaronitrile, éventuellement avec addition de 3-méthylpipéridine, est converti en présence d'un premier catalyseur avec de l'hydrogène en un mélange des méthyl-5-(3-méthylpipéridino)pentanenitriles isomères de formule générale 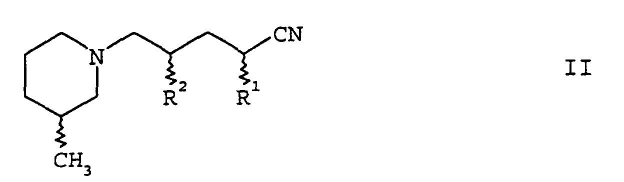 où R¹ et R² ont la signification citée ci-dessus, lequel, dans une seconde étape, est hydrogéné en présence d'un second catalyseur en un mélange des méthyl-5-(3-méthylpipéridino)pentanamines isomères des formules 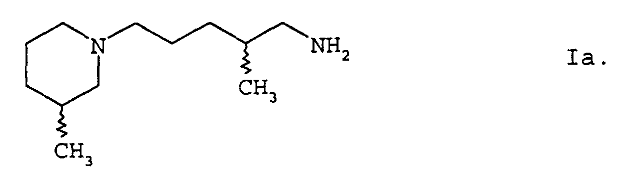 et 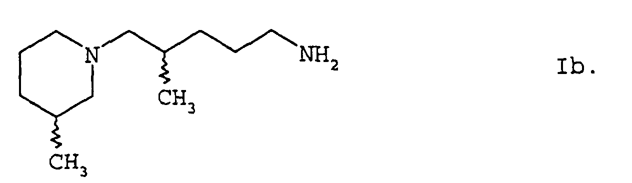 et éventuellement résolu en les deux isomères de structure.

2. Procédé selon la revendication 1 **caractérisé en ce qu'**un catalyseur palladium-support est utilisé comme premier catalyseur.

3. Procédé selon la revendication 2 **caractérisé en ce que** du palladium sur oxyde d'aluminium est utilisé comme catalyseur palladium-support.

4. Procédé selon la revendication 2 ou 3 **caractérisé en ce que** la réaction du 2-méthylglutaronitrile avec l'hydrogène est conduite à une température de 100 à 250°C et à une pression de 20 à 70 bar.

5. Procédé selon une ou plusieurs des revendications 1 à 4 **caractérisé en ce qu'**une quantité 1 à 5 fois molaire de 3-méthylpipéridine est ajoutée au 2-méthylglutaronitrile.

6. Procédé selon une ou plusieurs des revendications 1 à 5 **caractérisé en ce que** la réaction du 2-méthylglutaronitrile avec l'hydrogène est conduite en continu dans un réacteur à lit fixe.

7. Procédé selon une ou plusieurs des revendications 1 à 6 **caractérisé en ce que** l'hydrogénation des méthyl-5-(3-méthylpipéridino)pentanenitriles (II) est conduite en présence d'ammoniac.

8. Procédé selon une ou plusieurs des revendications 1 à 7 **caractérisé en ce qu'**un catalyseur rhodium-support est utilisé comme second catalyseur.

9. Procédé selon la revendication 8 **caractérisé en ce que** du rhodium sur charbon actif est utilisé comme catalyseur rhodium-support.

10. Procédé selon la revendication 8 ou 9 **caractérisé en ce que** l'hydrogénation des méthyl-5-(3-méthylpipéridino)pentanenitriles est conduite à une température de 5 à 100°C et à une pression de 1 à 50 bar.
